# EUROPÄISCHE PATENTSCHRIFT

(11) **EP 0 732 113 B1**
(45) Veröffentlichungstag und Bekanntmachung des Hinweises auf die Patenterteilung: **19.12.2001**
(21) Anmeldenummer: 96103912.0
(22) Anmeldetag: 13.03.1996
(51) Int. Cl.: A61M 5/142

(54) **Implantierbare Infusionspumpe**
Implantable infusion pump
Pompe de perfusion implantable

(30) Priorität: 17.03.1995 DE 19509634
(43) Veröffentlichungstag der Anmeldung: 18.09.1996
(73) Patentinhaber: Fresenius AG, 61350 Bad Homburg v.d.H (DE)
(72) Erfinder: Steinbach, Bernd, Dr., 61347 Bad Homburg v.d.H. (DE); Walter, Claus, 61348 Bad Homburg v.d.H. (DE)
(74) Vertreter: Luderschmidt, Schüler & Partner GbR

(56) Entgegenhaltungen:
- EP-A- 0 354 742
- EP-A- 0 732 112
- WO-A-83/02063
- DE-C- 4 432 991

## Beschreibung

Gegenstand der vorliegenden Erfindung ist eine implantierbare Infusionspumpe zur dosierten Abgabe von Medikamenten in den menschlichen Körper nach dem Oberbegriff des Patentanspruchs 1.

Implantierbare Infusionspumpen sind bereits bekannt. Die aus der DE 39 15 251 A1 bekannte Infusionspumpe weist eine Pumpenkammer auf, die durch ein schalenförmiges Kammerunterteil und ein damit verbundenes Kammeroberteil gebildet ist. Die Pumpenkammer ist durch eine flexible Membran in zwei Teilkammern getrennt. Die erste Teilkammer ist vom Kammeroberteil und der Membran begrenzt und als Medikamentenspeicher ausgebildet. Das Kammeroberteil enthält eine Nachfüllöffnung, die mit einem durchstechbaren Septum abgedichtet ist. Das Septum ist zwischen einem mit dem Kammeroberteilteil verbundenen Septenhalter und dem Kammeroberteil verklemmt. Der Medikamentenspeicher ist über eine Auslaßöffnung und gegebenenfalls eine Auslaßreduziereinrichtung mit einem Auslaßkatheter verbunden. Die zweite Teilkammer wird vom Kammerunterteil und der Membran begrenzt und ist als Druckkammer zur Aufnahme eines sich bei Körpertemperatur ausdehnenden Treibmittels vorgesehen.

Infusionspumpen mit ähnlichem Aufbau sind weiterhin aus der DE 40 38 049 A1, der DE 21 24 062 B2 sowie der DE 26 04 113 A1 bekannt. Eine weitere Infusionspumpe wird in der DE 44 32 991 A1 beschrieben, auf die hierin zu Offenbarungszwecken ausdrücklich Bezug genommen wird.

Die Pumpenbauteile bestehen bei diesen Infusionspumpen aus körperverträglichen Metall-Legierungen oder Kunststoffen, die durch Schweißverbindungen oder Rastverbindungen miteinander verbunden sind.

Implantierbare Infusionspumpen werden in einer subkutanen Tasche im Bereich des Abdomens des Patienten angeordnet, wobei die mit dem Septum verschlossene Nachfüllöffnung unter der Haut des Patienten tastbar ist. Der Medikamentenspeicher wird gefüllt, indem eine Spritze mit einer entsprechenden Kanüle durch die Haut des Patienten und durch das Septum gestochen wird und das Medikament bzw. die medizinische Lösung aufgrund des Spritzendrucks durch die Kanüle in den Medikamentenspeicher fließt.

Implantierbare Pumpen werden zur kontinuierlichen Medikation (gleichbleibende Dosis) über lange Zeiträume bei Patienten eingesetzt, die sonst nur durch Injizieren der Medikamente, z.B. Morphine, Heparine und dergleichen, mehrmals täglich behandelt werden können. Diese Pumpen haben gegenüber den üblichen Injektionen oder Infusionen den Vorteil, daß die verabreichte Dosis nicht mehr so weit überdosiert werden muß, daß trotz des Abbaus des Medikamentes bis zum nächsten Verabreichungszeitpunkt eine gewisse Mindestdosis nicht unterschritten wird, sondern eine gleichmäßige und insgesamt wesentlich verringerte Zufuhr des Medikamentes verwirklicht werden kann. An solche Infusionspumpen müssen, insbesondere bei Gabe von Schmerzmitteln, hohe Sicherheitsanforderungen gestellt werden. Eine eventuelle Überdosierung muß vermieden werden. Eine Überdosierung kann abhängig vom verabreichten Medikament zu einem hohen Gesundheitsrisiko führen, insbesondere bei Schmerzmitteln sogar tödlich wirken.

Die Wirkungsweise einer implantierbaren Infusionspumpe ist im wesentlichen der Art, daß das in der Pumpe enthaltene Treibmittel, dessen Siedepunkt unterhalb der Körpertemperatur liegt, nach Implantierung der Pumpe im Körper des Patienten teilweise verdampft und über die Trennwand, wie eine flexible Membran oder einen Faltenbalg, Druck auf das im Medikamentenspeicher befindliche Medikament bzw. die medizinische Lösung ausübt, worauf das Medikament bzw. die medizinische Lösung über ein Reduziersystem und einen Katheter zum Zielorgan fließt. Dabei verkleinert sich der Medikamentenraum unter Vergrößerung des Treibgasraumes. Die Volumenvergrößerung des Treibgasraumes wird von einer weiteren Verdampfung des Treibmittels aufgefangen und geschieht bei konstanter Temperatur so lange isobar, als das Treibmittel als Zweiphasensystem vorliegt und keine Fremdgase im Treibgasraum existieren. Die isobare Druckentwicklung ist für die Systeme zur Beibehaltung einer konstanten Förderrate notwendig.

Die bisher in implantierbaren Infusionspumpen als Trennwand angewandten Faltenbälge aus nichtrostendem Metall, wie z.B. Titan, sind sehr aufwendig. Andererseits können bisher als Trennwände Kunststofffolien, die erheblich kostengünstiger sind, wie Untersuchungen gezeigt haben, eine Diffusion des Treibgases aus dem Treibmittelraum in den Medikamentenraum und eine Diffusion von Gasen und Wasserdampf aus dem Medikamentenraum in den Treibmittelraum nicht verhindern. Ferner verhalten sich chlorierte Fluorkohlenwasserstoffe, die üblicherweise als Treibmittel angewandt werden, gegenüber Kunststoffen äußerst aggressiv und vermindern so deren Stabilität.

Eine implantierbare Infusionspumpe mit einer durch eine Membran in zwei Kammern unterteilten Pumpenkammer ist aus der WO 83/02063 bekannt. Die Membran der bekannten Infusionspumpe besteht aus einem metallbeschichtetem Elastomer.

Aufgabe der vorliegenden Erfindung ist es, eine implantierbare Infusionspumpe bereitzustellen, mit welcher eine Gasdiffusion (Treibgas einerseits und Gase und Wasserdampf andererseits) durch die Trennwand zwischen Medikamentenspeicher und Treibmittel enthaltender Druckkammer im wesentlichen ausgeschaltet ist und eine konstante Förderrate des Medikamentes über den gesamten Betriebszeitraum gewährleistet wird, die medikamentenverträglich, leicht mit Medikamenten zu befüllen, leicht und sicher zu handhaben ist, aus körperverträglichen Materialien und miteinander verträglichen Materialien besteht und einfach und kostengünstig herstellbar ist.

Diese Aufgabe wird bei einer Infusionspumpe der eingangs genannten Art dadurch gelöst, daß erfindungsgemäß die gasundurchlässige flexible Trennwand eine der Kontur der Innenform des Kammeroberteils folgend gewölbte Metallfolie ist.

Die erfindungsgemäß als Trennwand angewandte Metallfolie ist der Kontur der Innenform des Kammeroberteils (und entsprechend des Kammerunterteils) folgend gewölbt und flexibel und spannungsfrei. Diese gewölbte bzw. kuppelartige Ausbildung der Folie kann in jeder geeigneten Weise, z.B. in einem Tiefziehverfahren erreicht werden, in welchem die Folie der Innenform des Kammeroberteils (und entsprechend des Kammerunterteils) des Gehäuses der Infusionspumpe angepaßt wird.

Die Metallfolie kann eine Folie aus jedem für die vorliegenden Zwecke geeigneten Metall bestehen, wobei Folien aus Aluminium, Gold, Silber, Titan oder Platin bevorzugt sind. Insbesondere bevorzugt ist eine Aluminiumfolie. Die Dicke der Folie muß so gestaltet sein, daß einmal die Sperrwirkung gewährleistet ist und zum anderen ein Knicken der Folie vermieden wird. Die Dicke der Metallfolie liegt geeigneterweise im Bereich von 1-100 µm, vorzugsweise im Bereich von 3-40 µm und insbesondere im Bereich von 5-20 µm.

Gemäß einer ersten alternativen Ausführungsform ist die Metallfolie auf der dem Medikament bzw. der medizinischen Lösung zugewandten Seite mit einer polymeren Stützfolie kaschiert. Geeigneterweise kann Polyurethan als Kleber verwendet werden. Für die Stützfolie geeignet sind polymere Materialien, die eine gute Medikamentenverträglichkeit besitzen und eine gute Sperrwirkung gegen Wasserdampf, Sauerstoff und CO₂ aufweisen. Bevorzugt sind erfindungsgemäß Folien aus Polyethylenterephthalat. Die Dicke dieser Folien liegt geeigneterweise im Bereich von 1-100 µm, vorzugsweise 30-40 µm und insbesondere 5-20 um und kann beispielsweise 12 µm betragen.

Gemäß einer zweiten alternativen Ausführungsform ist die Metallfolie auf der dem Treibmittel zugewandten Seite mit einer polymeren Stützfolie kaschiert, wobei z.B. geeigneterweise Polyurethan als Kleber verwendet werden kann. Als polymere Materialien geeignet sind solche, die mit dem Treibmittel verträglich sind und eine gute Sperrwirkung gegen das Treibgas aufweisen. Geeignet sind erfindungsgemäß beispielsweise Folien aus Polyolefinen oder Polyamiden, wobei solche aus Polyolefinen bevorzugt sind. Von Polyolefinfolien bevorzugt sind Folien aus Polyethylen oder Polypropylen, wobei Folien aus Polyethylen besonders bevorzugt angewandt werden. Die Dicke dieser Folien beträgt geeigneterweise 2-400 µm, vorzugsweise 10-100 µm, insbesondere 25-100 µm und beispielsweise 70 µm.

Die Metallfolie kann aber auch zu beiden Seiten mit einer polymeren Stützfolie kaschiert sein, wobei die vorstehend genannten Folien jeweils auf der entsprechenden Seite der Metallfolie angeordnet sind. So kann die Metallfolie auf der der medizinischen Lösung zugewandten Seite mit einer Folie aus Polyethylenterephthalat und auf der dem Treibmittel zugewandten Seite mit einer Polyolefinfolie, wie einer Polyethylenfolie kaschiert sein. Ein solches Verbundsystem kann - vom medizinischen Speicher aus gesehen - vorzugsweise folgendermaßen aufgebaut sein:
a) Folie aus Polyethylenterephthalat, Dicke 12 µm
b) Metallfolie, vorzugsweise Aluminiumfolie, Dicke 12 µm
c) Polyethylenfolie, Dicke 70 µm.

Durch die polymeren Stützfolien werden die Biegeradien der Metallfolie erhöht und damit die Knickneigung herabgesetzt.

Gemäß einer weiteren Aussführungsform die Trennwand als Foliensystem ausgebildet sein, bei dem an der der medizinischen Lösung zugewandten Seite der vorstehend genannten Metallfolie oder Metallverbundfolie zusätzlich eine Polymerfolie mit guter Sperrwirkung gegen Wasserdampf und/oder eine Polymerfolie mit guter Medikamentenverträglichkeit und Sperrwirkung gegen Wasserdampf, Sauerstoff und CO₂ angeordnet ist, wobei, wenn beide zusätzlichen Folien vorliegen, die Polymerfolie mit guter Medikamentenverträglichkeit mit der medizinischen Lösung in Berührung kommt und die Polymerfolie mit guter Sperrwirkung gegen Wasserdampf zwischen der Polymerfolie mit guter Medikamentenverträglichkeit und der Metallfolie bzw. Metallverbundfolie angeordnet ist. Vorzugsweise liegen beide zusätzlichen Folien zur Metall- bzw. Metallverbundfolie vor.

Als Folie mit guter Medikamentenverträglichkeit und Sperrwirkung gegen Wasserdampf, Sauerstoff und CO₂ ist insbesondere eine Polyethylenterephthalatfolie geeignet. Diese Folien können eine Dicke im Bereich von 2-400 µm, vorzugsweise im Bereich von 25-250 µm, insbesondere eine Dicke im Bereich von 40-150 µm, z.B. eine Dicke von 100 µm besitzen. Als Folie mit guter Sperrwirkung gegen Wasserdampf ist jede Polymerfolie geeignet, die diese Eigenschaften besitzt, wobei eine Polychlortrifluorethylenfolie besonders bevorzugt ist. Die Foliendicke liegt geeigneterweise im Bereich von 2-400 µm, vorzugsweise im Bereich von 25-250 µm, insbesondere im Bereich von 40-150 µm und kann z.B. 127 µm betragen.

Die beiden zusätzlichen Folien können miteinander (falls beide vorhanden sind) und mit der Metallfolie bzw. Metallverbundfolie über die gesamte Fläche verbunden oder verklebt sein oder lediglich im Randbereich verbunden, z.B. randverschweißt oder verklebt sein, wobei die Randverbindung bevorzugt ist.

Ein besonders bevorzugtes Foliensystem weist - vom medizinischen Speicher aus gesehen - folgende Zusammensetzung auf:
a) Folie aus Polyethylenterephthalat
b) Folie aus Polychlortrifluorethylen
c) Verbundfolie aus
   i) Polyethylenterephthalatfolie
   ii) Metallfolie, vorzugsweise Aluminiumfolie
   iii) Polyolefinfolie, vorzugsweise Polyethylenfolie,
wobei die Folien in Dicken angewandt werden, wie sie vorstehend angegeben sind. Dieses Foliensystem ist ebenfalls der Kontur der Innenform des Kammeroberteils (und entsprechend des Kammerunterteils) folgend gewölbt, spannungsfrei und flexibel. Diese gewölbte Ausbildung des Foliensystems wird in der vorstehend bereits beschriebenen Weise erhalten.

Die angewandte Trennwand wird in üblicherweise in ihrem Randbereich zwischen die Randbereiche des Kammeroberteils und des Kammerunterteils sicher eingepreßt oder eingeklemmt.

Gemäß einer besonders bevorzugten Ausführungsform kann in der Infusionspumpe zusätzlich zu der vorstehend beschriebenen gasundurchlässigen Trennwand aus der Metallfolie bzw. Metallverbundfolie oder dem genannten Foliensystem zwischen dem Treibmittel und der Innenwand des Kammerunterteils eine Metallfolie oder eine Metallverbundfolie oder ein Metallfoliensystem, wie sie vorstehend beschrieben sind, angeordnet sein. Die Metallfolie bzw. Metallverbundfolie oder das Foliensystem ist der Kontur der Innenform des Kammerunterteils folgend gewölbt, wobei diese gewölbte Ausbildung in der vorstehend bereits beschriebenen Weise erzielt werden kann. Diese weitere Metallfolie bzw. Metallverbundfolie oder das weitere Foliensystem ist mit der erfindungsgemäß eingesetzten flexiblen gasundurchlässigen Trennwand im Randbereich entlang des gesamten Umfangs z.B. durch Randverschweißung oder -verklebung verbunden. Das auf diese Weise entstehende "pillenförmige Gebilde" dient als Druckkammer zur Aufnahme des Treibmittels. Dieser pillenförmige Druckraum ist mit seinem äußeren Randbereich entlang seines Umfangs - wie vorstehend für die Trennwand bereits beschrieben - zwischen die Randbereiche des Kammeroberteils und des Kammerunterteils sicher eingeklemmt oder eingepreßt.

Die Abdichtung des Druckraums am Umfang kann in geeigneter Weise erfolgen, wie z.B. über Dichtringe aus geeignetem Material, wie Elastomeren, z.B. Silikon-O-Ringen, oder Metallen oder durch Verkleben mit geeigneten Klebern, z.B. 2-Komponentenharzen.

Die Befüllung der Druckkammer mit dem Treibmittel kann nach Entfernung der vorhandenen Luft (z.B. durch Absaugen) in jeder geeigneten Weise erfolgen. Z.B. kann durch die Dichtfläche eine Mikrokapillare eingeführt werden, durch welche das Treibmittel in den Druckraum eingefüllt wird. Nach Befüllen mit dem Treibmittel kann dann die Kapillare vergossen oder herausgezogen werden.

Als Treibmittel können alle üblichen Treibmittel, wie sie in implantierbaren Infusionspumpen angewandt werden, eingesetzt werden. Vorzugsweise wird erfindungsgemäß als Treibmittel Hexafluorbutan angewandt, wobei 1,1,1,4,4,4-Hexafluorbutan besonders bevorzugt wird. Implantierbare Infusionspumpen, die diese Treibmittel enthalten, sind in der gleichzeitig mit der vorliegenden Anmeldung eingereichten Patentanmeldung mit dem Aktenzeichen P 195 09 632.0-35 mit dem Titel "Implantierbare Infusionspumpe" (unser Zeichen FR2664) beschrieben.

Die implantierbare Infusionspumpe, die die vorstehend beschriebene flexible gasundurchlässige Trennwand bzw. die "pillenförmig" ausgebildete Druckkammer enthalten, können in beliebiger geeigneter Weise ausgebildet und aus jedem geeigneten körperverträglichen Material, wie z.B. Metall oder Kunststoff hergestellt sein. Vorzugweise sind sie aus Kunststoff hergestellt, wobei Hartkunststoffe, wie Polysulfone (einschließlich Polyethersulfone), Polyamide und Polycarbonate, insbesondere Polysulfone bevorzugt sind. Die Verwendung von Infusionspumpen aus Kunststoff bringt erhebliche Vorteile. Abgesehen vom geringeren Gewicht sind bei Infusionspumpen aus Kunststoff die Voraussetzungen für die gleichzeitige Anwendung bestimmter therapeutischer oder diagnostischer Verfahren, wie Thermographie und NMR gegeben.

Insbesondere sind die Infusionspumpen so ausgebildet, wie sie in der DE 44 32 991 A1, beschrieben sind.

Bei der implantierbaren Infusionspumpe findet keine bzw. im wesentlichen keine Gasdiffusion durch die flexible Trennwand statt. Mit der erfindungsgemäßen Infusionspumpe wird über den gesamten Betriebszeitraum eine konstante Förderrate sichergestellt. Sie besitzt ein geringeres Gewicht, ist leicht zu befüllen, leicht und sicher zu handhaben und einfach und kostengünstig herstellbar.

Die Infusionspumpe gestattet bis zu maximal 120 Füllungen mit medizinischen Lösungen, wobei die Förderrate pro Tag z.B. 0,7, 1,0, 1,4 oder 2,0 ml betragen und der Medikamentenspeicher z.B. 30 ml der medizinischen Lösung enthalten kann. Die eingefüllte Treibmittelmenge ist gering und kann z.B. 2 ml betragen. Das Innenvolumen der Druckkammer kann z.B. 40 ml ausmachen.

Die Infusionspumpe ist zur kontinuierlichen dosierten Abgabe von Medikamenten wie z.B. Heparin, künstlichem Pankreasinsulin, Chemotherapeutika, Schmerzmittel, wie Morphium, Muskelrelaxantien, wie z.B. Baclofen, und dergleichen geeignet.

Im folgenden werden zwei Ausführungsbeispiele der Erfindung unter Bezugnahme auf die Zeichnungen näher erläutert.

Es zeigen:
- Figur 1: einen vertikalen Schnitt durch ein erstes Ausführungsbeispiel der Infusionspumpe in schematischer Darstellung,
- Figur 2: einen Vertikalschnitt durch ein zweites Ausführungsbeispiel der Infusionspumpe und
- Figur 3: einen Vertikalschnitt durch eine speziell gestaltete Infusionspumpe zur dosierten Abgabe von Medikamenten in den menschlichen Körper.

Die Infusionspumpe 1 ist ein scheibenförmiger rotationssymmetrischer Körper aus Hartkunststoff mit einer Pumpenkammer, die durch ein Kammerunterteil 2 und ein Kammeroberteil 3 gebildet ist und durch die flexible gasundurchlässige Trennwand 6 in zwei Teilkammern 7 und 8 getrennt ist. Die erste Teilkammer 7 dient als Medikamentenspeicher und die zweite Teilkammer 8 dient als Druckkammer zur Aufnahme des Treibmittels.

Die Nachfüllöffnung 12 ist durch ein durchstechbares Zentralseptum 13 dicht abgedeckt und weist unter dem Zentralseptum 13 einen Nachfüllraum mit einer festen Platte als Nadelstopp und Durchtrittsöffnungen zum Medikamentenspeicher 7 auf.

Die flexible gasundurchlässige Trennwand 6 ist der Kontur der Innenform des Kammeroberteils 3 (und entsprechend des Kammerunterteils 2) folgend gewölbt, was durch Tiefziehen erreicht wurde. Sie ist spannungsfrei. Diese Trennwand 6 besteht aus einem Foliensystem, das - vom Medikamentenspeicher 7 aus gesehen - folgendermaßen zusammengesetzt ist:
a) Folie aus Polyethylenterephthalat, Dicke 100 µm
b) Folie aus Polychlortrifluorethylen, Dicke 127 µm
c) Aluminiumverbundfolie aus
   i) Polyethylenterephthalatfolie, Dicke 12 µm
   ii) Aluminiumfolie, Dicke 12 µm
   iii) Polyethylenfolie, Dicke 70 µm.

Die Folien der Aluminiumverbundfolie sind mittels eines Polyurethanklebers miteinander verklebt. Die Folien a) und b) sind lediglich im Randbereich entlang des gesamten Umfangs miteinander und mit der Aluminiumverbundfolie verbunden. Die Trennwand 6 ist mit ihrem äußeren Randbereich entlang ihres gesamten Umfangs zwischen die Randbereiche des Kammeroberteils 3 und des Kammerunterteils 2 eingeklemmt bzw. eingepreßt.

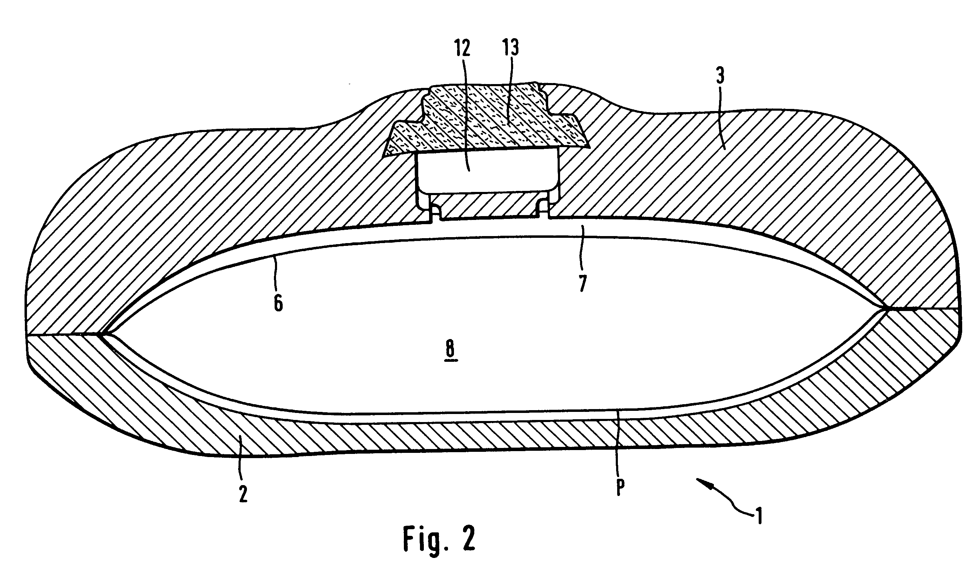

Die Figur 2 stellt ebenfalls einen Vertikalschnitt durch eine weitere schematisch veranschaulichte erfindungsgemäße Infusionspumpe dar.

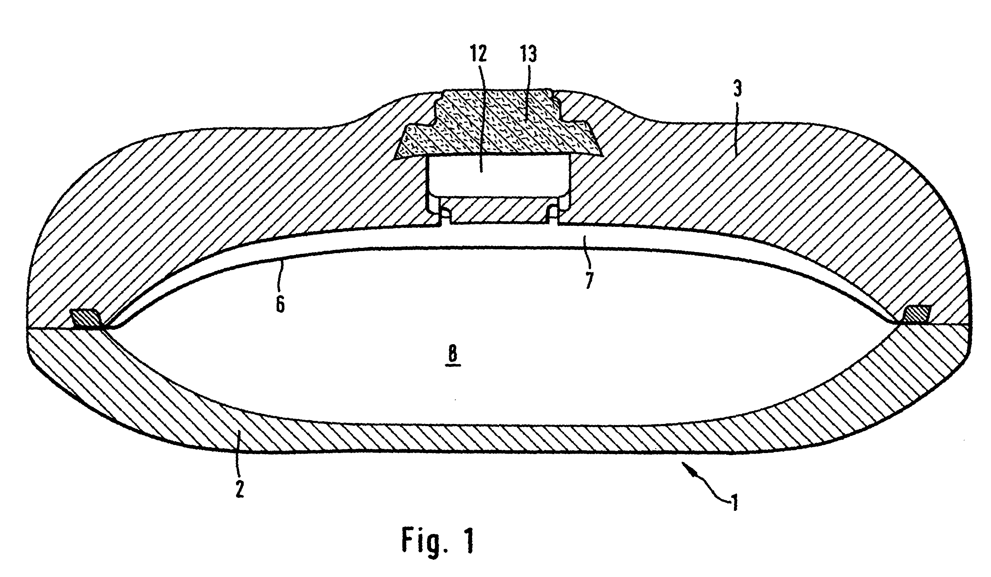

Diese Infusionspumpe entspricht der in Figur 1 dargestellten Infusionspumpe gemäß Beispiel 1 mit der Ausnahme, daß zusätzlich zu der Trennwand 6 eine ebenfalls gewölbte Metallverbundfolie P angeordnet ist. Diese Metallverbundfolie P ist durch Tiefziehen der Kontur der Innenwand des Kammerunterteils 2 folgend gewölbt und an den Rändern entlang des gesamten Umfangs mit der Trennwand 6, die die gleiche Zusammensetzung wie die gemäß Beispiel 1 beschriebene Trennwand besitzt, verbunden. Die Metallverbundfolie P besitzt - vom Druckraum 8 her gesehen - die folgende Zusammensetzung:
i) Polyethylenfolie, Dicke 70 µm
ii) Aluminiumfolie, Dicke 12 µm
iii) Polyethylenterephthalatfolie, Dicke 12 µm.

Aus der Trennwand 6 und der Metallverbundfolie P wird gemäß diesem Beispiel eine pillenförmige Druckkammer 8 für das Treibmittel gebildet. Diese Druckkammer ist, wie in Beispiel 1 beschrieben, mit ihrem Randbereich entlang des gesamten Umfangs zwischen die Randbereiche des Kammeroberteils 3 und des Kammerunterteils 2 eingeklemmt oder eingepreßt.

Als Treibmittel wird 1,1,1,4,4,4-Hexafluorbutan verwendet, das auf die Weise in die Druckkammer gebracht wurde, daß man durch die Dichtfläche im Randbereich eine Mikrokapillare einführte, die Luft aus der Druckkammer entfernte und durch die Mikrokapillare das Treibmittel in den Druckraum einfüllte. Die eingefüllte Menge betrug etwa 2 ml.

Das Innenvolumen des Druckraumes 8 beträgt etwa 40 ml, der Medikamentenspeicher 7 kann etwa 30 ml der medizinischen Lösung aufnehmen.

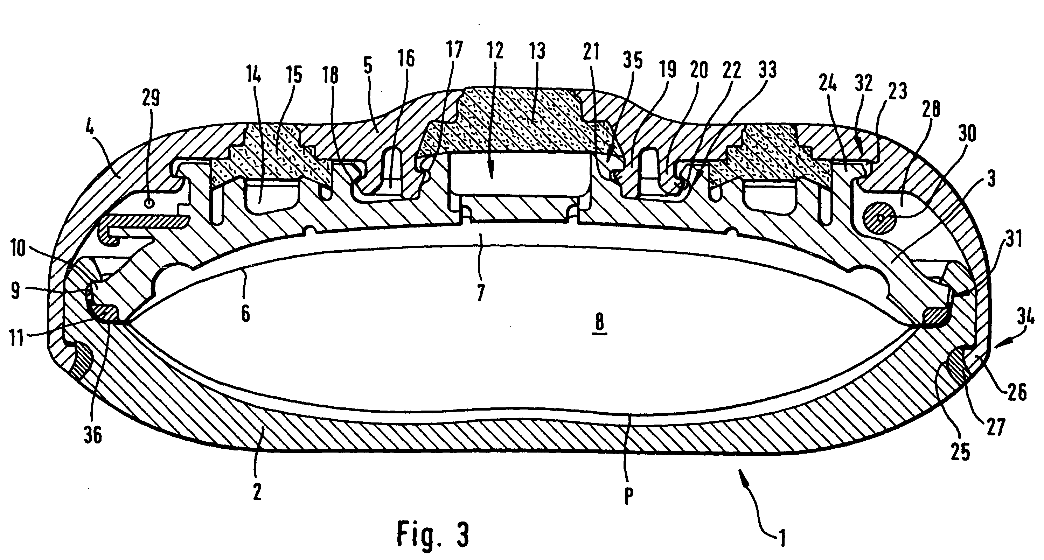

In Figur 3 ist ein Vertikalschnitt durch eine speziell gestaltete Infusionspumpe zur dosierten Abgabe von Medikamenten in den menschlichen Körper dargestellt.

Die Infusionspumpe 1 ist ein scheibenförmiger rotationssymmetrischer Körper aus Polysufon, die ein Gehäuse aufweist, das aus einem schalenförmigen Kammerunterteil 2, einem schalenförmig mit entgegengesetzter Wölbung ausgeführten Kammeroberteil 3, einem Überwurfteil 4 und einem Septenhalter zusammengesetzt ist. Der Innenraum ist durch eine gewölbte Trennwand 6 und eine ebenfalls gewölbte Aluminiumverbundfolie P in eine erste Teilkammer, die als Medikamentenspeicher 7 dient, und eine zweite Teilkammer, die als Druckkammer 8 zur Aufnahme des Treibmittels dient, geteilt. Die Druckkammer 8 wird durch Randverschweißung oder -verklebung der Trennwand 6 und der Aluminiumverbundfolie P gebildet und ist pillenförmig gestaltet. Am oberen inneren Randbereich des Kammerunterteils 2 ist eine Ringnut 9 eingeformt, in die in einer Rastverbindung ein umlaufender zugeordneter Rand 10 des Kammeroberteils 3 zusammen mit einem O-Ring 11 und einem Rand 36, der aus der Trennwand 6 und der Aluminiumverbundfolie P gebildet wird, eingerastet und dicht gehalten ist.

Eine Nachfüllöffnung 12 ist durch ein scheibenförmiges und durchstechbares Zentralseptum 13 dicht abgedeckt, wobei die Nachfüllöffnung 12 einen Nachfüllraum unter dem Zentralseptum 13 mit einer festen Platte als Nadelstopp und Durchtrittsöffnungen zum Medikamentenspeicher 7 aufweist.

Weiter ist an der Oberseite des Kammeroberteils 3 eine konzentrische Ringkammer 14 eingeformt, die von einem Ringseptum 15 dicht abgedeckt ist.

Zwischen der Ringkammer 14 und der Nachfüllöffnung 12 ist eine konzentrische Ringnut 16 eingeformt, an deren innerer und äußerer Seitenwand jeweils eine umlaufene Rastnut 17, 18 angebracht ist. Der ringförmige Septenhalter 5 übergreift mit seinem inneren Rand den Rand des scheibenförmigen Zentralseptums 13 und mit seinem äußeren Rand den ringinneren Rand des Ringseptums 15. Zudem greift der Septenhalter 5 mit nebeneinander liegenden Ringstegen 19, 20 in die Ringnut 16 ein, wobei Rastnasen 21, 22 auf den Ringstegen 19, 20 in die Rastnuten 17, 18 eingreifen.

Der ringäußere Rand des Ringseptums 15 ist in einer stufenförmigen Anordnung von einem entsprechend geformten Randbereich des Überwurfteils 4 überdeckt und gegen das Kammeroberteil 3 dicht verklemmt. Dazu ist in diesem Randbereich des Überwurfteils 4 eine Rastverbindung zwischem dem Überwurfteil 4 und dem Kammeroberteil 3 vorgesehen, die aus einer umlaufenden Rastnut 23 am Überwurfteil 4 und einer umlaufenden Rastnase 24 am Kammeroberteil 3 besteht.

Am seitlichen äußeren Randbereich des Kammerunterteils 2 ist eine weitere Ringnut 25 eingeformt, in die eine umlaufende Rastnase 26 zusammen mit einem eingelegten O-Ring 27 eingreift. Die Rastnase 26 stellt den unteren Rand des Überwurfteils 4 dar, wobei dieses den seitlichen Bereich des Kammeroberteils 3 und den äußeren seitlichen Randbereich des Kammerunterteils 2 glockenförmig übergreift. Das Überwurfteil 4 stützt sich (bei großer Druckbelastung) am Kammeroberteil 3 über die Rastnasenanordnung 24 und über das Ringseptum 15 zum Kammerunterteil 2 hin ab.

Zwischen dem äußeren Wandbereich des Kammeroberteils 3 und einem inneren Wandbereich des Überwurfteils 4 ist ein Ringraum 28 zur Aufnahme einer Auslaßreduziereinrichtung 29 (hier schematisch als Schnitt durch eine Kapillare dargestellt) und eines Auslaßkatheters 30 gebildet.

Die Verbindung 31, gebildet aus der Ringnut 9, dem Rand 10 und dem O-Ring 11, die Verbindung 32, gebildet aus der Rastnut 23 und der Rastnase 24 sowie die Verbindung 33, bestehend aus der Rastnut 18 und den Rastnasen 22, stellen Hauptverbindungen dar, die bei normaler betriebsmäßiger Belastung die auftretenden Innendruckbelastungen im wesentlichen abstützen.

Die Verbindung 34, bestehend aus der Ringnut 25, der Rastnase 26 und dem O-Ring 27, sowie die Verbindung 35, bestehend aus der Rastnut 17 und den Rastnasen 21, sind Nebenverbindungen, die bei normaler betriebsmäßiger Belastung noch keine oder nur eine geringe Abstützfunktion haben. Erst bei einer Druckerhöhung über den normalen Betriebsdruck, insbesondere bei einem Versagen der Hauptverbindungen 31, 32, 33 übernehmen die Nebenverbindungen 34, 35 eine Abstützfunktion.

Die Hauptverbindungen 31, 32, 33 stehen dazu in dauerndem Eingriff und stellen die Integrität der implantierten Infusionspumpe unter Normalbedingungen sicher. Bei Erhöhung des Innendrucks entweder im Ringraum 28 oder im Medikamentenspeicher 7 kommen die Nebenverbindungen 34, 35 zunehmend in Eingriff. Dadurch werden die Spannungen von den Hauptverbindungen 31, 32, 33 abgeleitet und teilen sich auf Haupt- und Nebenverbindungen auf.

Sollte beispielsweise die Hauptverbindung 31 versagen, so wird die Verbindungsaufgabe von der Nebenverbindung 34 übernommen. Da gleichzeitig ein Spalt zwischen Kammeroberteil 3 und Kammerunterteil 2 entsteht, entweicht Medikament in den Ringraum 28 zwischen dem Überwurfteil 4 und dem Kammeroberteil 3, so daß auch dadurch Druck und damit die Belastung auf die Verbindung insgesamt abnimmt. Dadurch ist die Nebenverbindung 34 in der Lage, die Integrität der Infusionspumpe nach außen hin sicherzustellen. Wesentlich ist dabei, daß das Unterteil im oberen Stegbereich versagt, nicht aber im Unterbereich, was konstruktiv sichergestellt werden kann.

Bei einem Versagen der Hauptverbindung 32 zwischen dem Kammeroberteil 3 und dem Überwurfteil 4 wird die Verbindung ebenfalls durch die Nebenverbindung 34 übernommen.

Entsprechend wird bei einem Versagen oder einer Überlastung der Hauptverbindung 33 eine Übernahme oder Aufteilung auf die Nebenverbindung 35 übertragen.

In jedem Fall ergibt sich der Vorteil, daß bei einer Überlastung oder bei einem Versagen der Hauptverbindungen 31, 32, 33 Nebenverbindungen 34, 35 zur Verfügung stehen, die den sicheren Weiterbetrieb der Infusionspumpe 1 gewährleisten, wodurch die Sicherheit für einen Patienten erheblich verbessert ist.

Die in diesem Beispiel angewandte Trennwand 6 und die Aluminiumverbundfolie P entsprechen der in Beispiel 2 angewandten Trennwand 6 bzw. Aluminiumverbundfolie sowohl hinsichtlich Zusammensetzung als auch Gestalt. Der pillenförmige Druckraum 8 weist ein Innenvolumen von 40 ml auf und enthält etwa 2 ml 1,1,1,4,4,4-Hexafluorbutan als Treibmittel. Der Medikamentenspeicher kann bis zu 30 ml medizinische Lösung aufnehmen. Diese Infusionspumpe gestattet bis zu maximal 120 Füllungen mit der medizinischen Lösung und Förderraten pro Tag von z.B. 0,7, 1,0, 1,4 oder 2,0 ml der medizinischen Lösung.

## Patentansprüche

1. Implantierbare Infusionspumpe (1) zur dosierten Abgabe von Medikamenten in den menschlichen Körper, mit einer Pumpenkammer, die durch ein Kammerunterteil (2) und ein damit verbundenes Kammeroberteil (3) gebildet ist, wobei
a) die Pumpenkammer durch eine gasundurchlässige flexible Trennwand (6) in zwei Teilkammern getrennt ist,
b) die erste Teilkammer (7) vom Kammeroberteil (3) und der flexiblen Trennwand (6) begrenzt ist und als Speicher für medizinische Lösungen ausgebildet ist, das Kammeroberteil (3) eine Nachfüllöffnung (12) aufweist, die durch wenigstens ein durchstechbares Septum (13) abgedichtet ist, und der Speicher für medizinische Lösungen über eine Auslaßöffnung und gegebenenfalls eine Auslaßreduziereinrichtung (29) mit einem Auslaßkatheter (30) verbunden ist, und
c) die zweite Teilkammer (8) vom Kammerunterteil (2) und der flexiblen Trennwand (6) begrenzt ist und als Druckkammer zur Aufnahme eines Treibmittels ausgebildet ist,
**dadurch gekennzeichnet,**
**daß** die gasundurchlässige flexible Trennwand (6) eine der Kontur der Innenform des Kammeroberteils (3) folgend gewölbte Metallfolie ist, die auf der der medizinischen Lösung zugewandten Seite und/oder auf der dem Treibmittel zugewandten Seite mit einer polymeren Stützfolie kaschiert ist.

2. Infusionspumpe nach Anspruch 1, **dadurch gekennzeichnet, daß** die Metallfolie aus Aluminium, Gold, Silber, Titan oder Platin besteht.

3. Infusionspumpe nach Anspruch 2, **dadurch gekennzeichnet, daß** die Metallfolie eine Aluminiumfolie ist.

4. Infusionspumpe nach einem oder mehreren der Ansprüche 1 bis 3, **dadurch gekennzeichnet, daß** die dem Treibmittel zugewandte Stützfolie der Metallverbundfolie eine Polyolefinfolie ist.

5. Infusionspumpe nach Anspruch 4, **dadurch gekennzeichnet, daß** die Polyolefinfolie eine Polyethylenfolie ist.

6. Infüsionspumpe nach einem oder mehreren der Ansprüche 1 bis 5, **dadurch gekennzeichnet, daß** die der medizinischen Lösung zugewandte Stützfolie der Metallverbundfolie eine Folie aus Polyethylenterephthalat oder Polyamid ist.

7. Infusionspumpe nach Anspruch 6, **dadurch gekennzeichnet, daß** die Folie aus Polyethylenterephthalat besteht.

8. Infusionspumpe nach einem oder mehreren der Ansprüche 1 bis 7, **dadurch gekennzeichnet, daß** zusätzlich auf der der medizinischen Lösung zugewandten Seite der Metallfolie bzw. Metallverbundfolie eine Polymerfolie mit guter Sperrwirkung gegen Wasserdampf angeordnet ist.

9. Infusionspumpe nach Anspruch 8, **dadurch gekennzeichnet, daß** die Polymerfolie eine Polychlortrifluorethylen-Folie ist.

10. Infusionspumpe nach Anspruch 8 oder 9, **dadurch gekennzeichnet, daß** zusätzlich eine weitere, mit der medizinischen Lösung in Berührung kommende Polymerfolie Berührung kommende Polymerfolie mit guter Medikamentenverträglichkeit und Sperrwirkung gegen Wasserdampf, Sauerstoff und CO₂ angeordnet ist.

11. Infusionspumpe nach Anspruch 10, **dadurch gekennzeichnet, daß** die Polymerfolie eine Polyethylenterephthalat-Folie ist.

12. Infusionspumpe nach einem oder mehreren der Ansprüche 1 bis 11, **dadurch gekennzeichnet, daß** zusätzlich zwischen dem Treibmittel und der Innenwand des Kammerunterteils (2) eine der Innenwand dieses Kammerunterteils folgend gewölbte Metallfolie oder Metallverbundfolie (P) angeordnet ist, die mit der Trennwand (6) im Randbereich entlang des gesamten Umfangs verbunden ist und vom Druckraum (8) her gesehen die gleiche Zusammensetzung wie die Trennwand (6) gemäß Anspruch 1 aufweist.

13. Infusionspumpe nach einem oder mehreren der Ansprüche 1 bis 12, **dadurch gekennzeichnet, daß** das Treibmittel Hexafluorbutan ist.

14. Infusionspumpe nach Anspruch 13, **dadurch gekennzeichnet, daß** das Treibmittel 1,1,1,4,4,4-Hexafluorbutan ist.

## Claims

1. An implantable infusion pump (1) for the measured dispensing of medicaments into the human body, with a pump chamber constituted by a chamber bottom part (2) and, connected to it, a chamber upper part (3), whereby
a) the pump chamber is separated into two partial chambers by a flexible partition (6) which is impermeable to gas,
b) the first partial chamber (7) is bounded by the chamber upper Part (3) and the flexible partition (6) and is constructed as a means of storing medicinal solutions, the chamber upper part (3) has a topping-up aperture (12) which is sealed by at least one pierceable septum (13), and the means of storing medicinal solutions is connected to an outlet catheter (30) via an outlet orifice and possibly an outlet reducing means (29), and
c) the second chamber (8) is bounded by the chamber bottom part (2) and the flexible partition (6) and is constructed as a pressure chamber to accommodate a propellant, **characterised in that** the gas-impermeable flexible partition (6) is a curved metal film which follows the contours of the inner shape of the chamber upper part (3) and which is lined with a polymer bracing film on the side facing the medicinal solution and/or on the side facing the propellant.

2. An infusion pump according to claim 1, **characterised in that** the metal foil consists of aluminium, gold, silver, titanium or platinum.

3. An infusion pump according to claim 2, **characterised in that** the metal foil is an aluminium foil.

4. An infusion pump according to one or more of claims 1 to 3, **characterised in that** the bracing foil ofthe composite metal foil and which faces the propellant is a polyolefin film.

5. An infusion pump according to claim 4, **characterised in that** the polyolefin film is a polyethylene film.

6. An infusion pump according to one or more of claims 1 to 5, **characterised in that** the bracing foil of the composite metal foil on the side facing the medicinal solution is a film of polyethylene terephthalate or polyamide.

7. An infusion pump according to claim 6, **characterised in that** the film consists of polyethylene terephthalate.

8. An infusion pump according to one or more of claims 1 to 7, **characterised in that** on the side of the metal foil or composite metal foil which faces the medicinal solution there is also a polymer film which has a good barrier effect against water vapour.

9. An infusion pump according to claim 8, **characterised in that** the polymer film is a polychlorotrifluoroethylene film.

10. An infusion pump according to claim 8 or 9, **characterised in that** additionally, a further polymer film which has good medicament compatibility and a barrier effect against water vapour, oxygen and CO₂ is provided which comes in contact with the medicinal solution.

11. An infusion pump according to claim 10, **characterised in that** the polymer film is a polyethylene terephthalate film.

12. An infusion pump according to one or more of claims 1 to 11, **characterised in that** here is additionally between the propellant and the inside wall of the chamber bottom part (2) a metal foil or composite metal foil (P) which is curved to follow the inside wall of this chamber bottom part and which is over its total periphery connected to the marginal portion of the partition (6) and which, viewed from the pressure space (8), has the same composition as the partition (6) according to claim 1.

13. An infusion pump according to one or more of claims 1 to 12, **characterised in that** the propellant is hexafluorobutane.

14. An infusion pump according to claim 13, **characterised in that** the propellant is 1,1,1,4,4,4-hexafluorobutane.

## Revendications

1. Pompe de perfusion implantable (1) pour la délivrance dosée de médicaments dans les corps humains, ayant une chambre de pompe, qui est formée d'une partie inférieure de chambre (2) et d'une partie supérieure de chambre (3) qui est reliée à celle-ci, dans laquelle
a) la chambre de pompe est séparée en deux chambres partielles par une paroi de séparation (6) flexible imperméable aux gaz,
b) la première chambre partielle (7) est délimitée par la partie supérieure de la chambre (3) et la paroi de séparation flexible (6) et est construite comme réserve pour des solutions médicinales, la partie supérieure de la chambre (3) présente une ouverture de remplissage (12), qui est fermée de manière étanche par au moins un septum perforable (13), et la réserve pour les solutions médicinales est reliée à travers une ouverture de sortie et éventuellement un dispositif de réduction de la sortie (29) avec un cathéter de sortie (30), et
c) la deuxième chambre partielle (8) est délimitée par la partie inférieure de la chambre (2) et la paroi de séparation flexible (6) et est construite comme chambre de pression pour recevoir un agent propulseur,
**caractérisée en ce que**,
la paroi de séparation (6) imperméable aux gaz flexible est une feuille métallique cintrée en suivant le contour de la forme intérieure de la partie supérieure de la chambre (3), qui est doublée sur la face tournée vers la solution médicinale et/ou sur la face tournée vers l'agent propulseur avec une feuille de protection polymère.

2. Pompe de perfusion selon la revendication 1, **caractérisée en ce que** la feuille métallique est constituée d'aluminium, d'or, de titane ou de platine.

3. Pompe de perfusion selon la revendication 2, **caractérisée en ce que** la feuille métallique est une feuille d'aluminium.

4. Pompe de perfusion selon une ou plusieurs des revendications 1 à 3, **caractérisée en ce que** la feuille de protection de la feuille de composite métallique tournée vers l'agent propulseur est une feuille de polyoléfine.

5. Pompe de perfusion selon la revendication 4, **caractérisée en ce que** la feuille de polyoléfine est une feuille de polyéthylène.

6. Pompe de perfusion selon une ou plusieurs des revendications 1 à 5, **caractérisée en ce que** la feuille de protection de la feuille de composite métallique tournée vers la solution médicinale est une feuille de polytéréphtalate d'éthylène ou de polyamide.

7. Pompe de perfusion selon la revendication 6, **caractérisée en ce que** la feuille est constituée de polytéréphtalate d'éthylène.

8. Pompe de perfusion selon une ou plusieurs des revendications 1 à 7, **caractérisée en ce qu'**en plus on dispose sur la face de la feuille métallique ou de la feuille de composite métallique tournée vers la solution médicinale une feuille de polymère ayant une bonne efficacité comme barrière contre la vapeur d'eau.

9. Pompe de perfusion selon la revendication 8, **caractérisée en ce que** la feuille de polymère est une feuille de polychlorotrifluoroéthylène.

10. Pompe de perfusion selon la revendication 8 ou 9, **caractérisée en ce qu'**en plus on dispose une autre feuille de polymère venant en contact avec la solution médicinale ayant une bonne compatibilité avec le médicament et une bonne efficacité comme barrière contre la vapeur d'eau, l'oxygène et le CO₂.

11. Pompe de perfusion selon la revendication 10, **caractérisée en ce que** la feuille de polymère est une feuille de polytéréphtalate d'éthylène.

12. Pompe de perfusion selon une ou plusieurs des revendications 1 à 11, **caractérisée en ce qu'**en plus on dispose entre l'agent propulseur et la paroi interne de la partie inférieure de la chambre (2) une feuille métallique ou une feuille métallique composite (P) cintrée en suivant la paroi intérieure de cette partie inférieure de chambre, qui est liée avec la paroi de séparation (6) dans la zone du contour le long de l'ensemble du périmètre et vue de la chambre de pression (8) présente la même composition que la paroi de séparation (6) selon la revendication 1.

13. Pompe de perfusion selon une ou plusieurs des revendications 1 à 12, **caractérisée en ce** l'agent propulseur est l'hexafluorobutane.

14. Pompe de perfusion selon la revendications 13, **caractérisée en ce** l'agent propulseur est le 1,1,1,4,4,4-hexafluorobutane.
